Europäisches Patentamt

European Patent Office

Office européen des brevets

(19)

(11) Veröffentlichungsnummer: **0 046 288**
**B1**

(12) **EUROPÄISCHE PATENTSCHRIFT**

(45) Veröffentlichungstag der Patentschrift:
**15.02.84**

(21) Anmeldenummer: **81106359.3**

(22) Anmeldetag: **17.08.81**

(51) Int. Cl.³: **C 07 C 95/02**

(54) **Verfahren zur Herstellung von 3-Dimethylamino-2,2-dimethylpropanal.**

(30) Priorität: **19.08.80 DE 3031248**

(43) Veröffentlichungstag der Anmeldung:
**24.02.82 Patentblatt 82/8**

(45) Bekanntmachung des Hinweises auf die Patenterteilung:
**15.02.84 Patentblatt 84/7**

(84) Benannte Vertragsstaaten:
**CH DE FR GB IT LI SE**

(56) Entgegenhaltungen:
**CH - A - 147 156**

**CHEMICAL ABSTRACTS, Band 73, Nr. 5, 3. August 1970, Seite 358, Nr. 25374v, Columbus, Ohio, U.S.A. A.V. BOGATSKII et al.: "Synthesis of some alkylamino-substituted 1,3-dioxanes"**
**JOURNAL OF THE CHEMICAL SOCIETY, Mai 1958, Seiten 1967-1974, C.B. CLARKE et al.: "Decahydro-isoquinolines and related compounds. Part II. Some further examples of abnormal ultraviolet absorption"**

(73) Patentinhaber: **Ruhrchemie Aktiengesellschaft, Bruchstrasse 219, D-4200 Oberhausen 13 (DE)**

(72) Erfinder: **Bernhagen, Wolfgang, Dr. Dipl.-Chem., Froschheide 19, D-4330 Mülheim/Ruhr (DE)**
Erfinder: **Falk, Volker, Dr. Dipl.-Chem., Lützowstrasse 36, D-4200 Oberhausen 13 (DE)**
Erfinder: **Springer, Helmut, Drostenkampstrasse 24, D-4200 Oberhausen 13 (DE)**
Erfinder: **Weber, Jürgen, Dr. Dipl.-Chem., Bunsenstrasse 17, D-4200 Oberhausen 13 (DE)**
Erfinder: **Wiebus, Ernst,, Ferdinandstrasse 77, D-4200 Oberhausen 14 (DE)**
Erfinder: **Kniep, Claus, Rosenstrasse 93, D-4200 Oberhausen 1 (DE)**

(74) Vertreter: **Reichelt, Karl-Heinz, Dr., m. Br. Ruhrchemie Aktiengesellschaft Abt. PLD Postfach 13 01 60, D-4200 Oberhausen 13 (DE)**

### Verfahren zur Herstellung von 3-Dimethylamino-2,2-dimethylpropanal

Die vorliegende Erfindung betrifft ein Verfahren zur Herstellung von 3-Dimethylamino-2,2-dimethylpropanal aus Isobutyraldehyd, Formaldehyd und Dimethylamin.

Aminoaldehyde, die sich von der Neopentyl-(d.h. 2,2-Dimethylpropyl-)Struktur ableiten, besitzen grosses Interesse als Zwischenprodukte für die Herstellung von Pharmazeutika, Pflanzenwachstumshormonen, Fungiziden, Herbiziden und Flotationshilfsmitteln. Besondere Bedeutung haben Aminoaldehyde, die neben der mittelständigen Verzweigung des Kohlenstoffgerüstes eine tertiäre Aminogruppe in 3-Stellung enthalten. Diese Verbindungen werden z.B. zur Herstellung von 3-Aminopyridin-Derivaten, 1,2-Dihalopyridazinen, Benzo-1,3-dioxanen, substituierten Imidazolen und Amidinen verwendet.

Es ist bekannt, Alkylaminoaldehyde durch Kondensation von CH-aciden Verbindungen mit Formaldehyd und Ammoniak oder Aminen herzustellen [Mannich et al. in Berichte 65, 387 (1932)]. Diese Umsetzung, sie lässt sich als Spezialfall in den Rahmen des umfassenden Reaktionstypus der Aminomethylierung einordnen, wird als Mannich-Reaktion bezeichnet.

Zur Darstellung von 3-Dimethylamino-2,2-dimethylpropanal nach der Mannich-Reaktion geht man von Isobutyraldehyd, Paraformaldehyd und Dimethylaminhydrochlorid aus (Mannich, 1.c.). Ein wesentliches Merkmal dieser Arbeitsweise ist die Gegenwart einer Säure, die dem Reaktionsmedium entweder als solche oder in Form des Aminsalzes zugesetzt wird. Die Kondensation verläuft also im saurem Milieu.

Die Notwendigkeit bei pH-Werten unter 7 zu arbeiten, hat erhebliche Nachteile, die zu grossen Schwierigkeiten bei der technischen Herstellung der Verbindung führt. Neben der Mannich-Reaktion läuft im sauren Medium nämlich auch eine Trimerisierung des Isobutyraldehyds zu 2, 4, 6-Triisopropyl-1, 3, 5-trioxan, wodurch die Ausbeute an dem erwünschten Aminoaldehyd erheblich vermindert wird. Bei Verwendung von Salzsäure ist der Einsatz halogenbeständiger Reaktoren erforderlich.

Darüber hinaus fällt die Mannichbase als Salz an, aus dem, über eine sehr aufwendige, kostspielige Aufarbeitung, die teilweise mit umweltbelastenden Faktoren verbunden ist, der Aminoaldehyd gewonnen werden muss. So bestand daher die Aufgabe ein Verfahren zu entwickeln, das die aufgezeigten Nachteile vermeidet und die Herstellung von 3-Dimethylamino-2,2-dimethylpropanal auch im technischen Massstab auf einfache Weise erlaubt.

Überraschenderweise wurde nun gefunden, dass man 3-Dimethylamino-2,2-dimethylpropanal aus Isobutyraldehyd, Dimethylamin und Formaldehyd bei 80 bis 120°C erhält, wenn die Umsetzung bei pH-Werten von 9 bis 11 erfolgt.

Die Ausgangsstoffe werden im molaren Verhältnis eingesetzt, jedoch schadet ein Überschuss von Dimethylamin nicht. Isobutyraldehyd und Dimethylamin finden in ihrer handelsüblichen Form Anwendung. Formaldehyd kann gasförmig, als wässrige Lösung oder als Polymeres wie Paraformaldehyd oder Trioxymethylen zur Reaktion gebracht werden. Die Anwesenheit eines Lösungsmittels für die Ausgangsstoffe und/oder das Reaktionsprodukt ist nicht erforderlich, die Anwesenheit von Wasser – bei Verwendung von wässriger Formaldehydlösung – stört nicht.

Der für das erfindungsgemässe Verfahren charakteristische pH-Bereich stellt sich im Reaktionsmedium aufgrund der Anwesenheit des Amins ein. Besonders zweckmässig ist es bei pH-Werten von 9 bis 11 zu arbeiten. Um sie zu erreichen, muss gegebenenfalls überschüssiges Amin angewandt werden.

Die Umsetzung wird absatzweise in einem Druckgefäss durchgeführt, in dem Isobutyraldehyd und Formaldehyd vorgelegt und mit dem als Gas eingeleiteten Dimethylamin vermischt werden. Unter Rühren heizt man auf 80 bis 120°C auf, wobei sich ein Druck von 1,5 bis 4 bar einstellt. Die Reaktionszeit beträgt je nach angewandter Temperatur 1 bis 6 Stunden. Nach einer kontinuierlichen Arbeitsweise führt man die Ausgangsstoffe getrennt einem Strömungsrohr zu und lässt sie bei einer Verweilzeit von 0,5 bis 3 h reagieren.

Zur Aufarbeitung wird das Reaktionsgemisch destilliert. Bei Verwendung wässriger Formaldehyldlösung bilden sich eine wässrige und eine organische Phase, die voneinander getrennt werden. Aus der organischen Phase kann man anschliessend das 3-Dimethylamino-2,2-dimethylpropanal isolieren.

In den folgenden Beispielen wird die Erfindung näher erläutert:

### Beispiel 1

In einem 5-l-Rührautoklaven werden 720 g (10 Mol) Isobutyraldehyd und 1000 g wässrige, 30%ige Formaldehydlösung (10 Mol) vorgelegt und mit 450 g gasförmigem Dimethylamin (10 Mol) vermischt. Das Gemisch, das einen pH-Wert von 11 aufweist, wird unter Rühren auf 100°C erhitzt und 5 Stunden bei dieser Temperatur belassen. Es stellt sich ein Eigendruck von 1,8 bis 2,5 bar ein. Nach Abschluss der Reaktionszeit lässt man abkühlen und entnimmt das Produkt dem Autoklaven. Es bildeten sich 1340 g einer organischen und 830 g Wasserphase, die getrennt werden. Durch Vakuumdestillation der organischen Phase an einer Kolonne mit 24 Böden erhält man 946 g 3-Dimethylamino-2,2-dimethylpropanal in einer Reinheit von mehr als 98% entsprechend einer Ausbeute von etwa 71,8% mit folgenden Kenndaten:

Dichte:     $d_4^{20}$     $-0,852$
Brechung:   $n_D^{20}$     $-1,4231$

Carbonylzahl             −440 mg KOH/g

Siedepunkt             −98°C / 150 Torr

**Beispiel 2**

In ein senkrecht stehendes Strömungsrohr werden über separate Leitungen stündlich 600 g Isobutyraldehyd, 833 g wässrige 30%ige Formaldehydlösung und 375 g Dimethylamin vom Boden her eingeleitet. Mit Stickstoff wird ein Druck von 20 bis 25 bar aufrecht erhalten. Bei einer Reaktionstemperatur von 100°C, und einer mittleren Verweilzeit von etwa 1 h erhält man am Kopf des Reaktors ein Reaktionsprodukt etwa folgender Zusammensetzung:

| | |
|---|---|
| Vorlaufkomponenten | 4,0 Gew.-% |
| Isobutyraldehyd | 0,9 Gew.-% |
| 3-Dimethylamino-2,2-dimethyl-propanal | 49,9 Gew.-% |
| höhersiedende Anteile | 4,7 Gew.-% |
| Wasser | 40,5 Gew.-% |

Danach beträgt die Selektivität der Umsetzung zum 3-Dimethylamino-2,2-dimethylpropanal etwa 84% der Theorie.

Durch nachfolgende Destillation wird, wie in Beispiel 1, ein Produkt mit einer Reinheit von etwa 98% erhalten.

**Patentanspruch**

1. Verfahren zur Herstellung von 3-Dimethylamino-2,2-dimethylpropanal aus Isobutyraldehyd, Dimethylamin und Formaldehyd bei 80 bis 120°C, dadurch gekennzeichnet, dass die Umsetzung bei einem pH-Wert von 9 bis 11 erfolgt.

**Claim**

1. Process for preparing 3-dimethylamino-2,2-dimethylpropanal from isobutyraldehyde, dimethylamine and formaldehyde at 80 to 120°C, characterised in that the reaction is carried out at a pH-value of 9 to 11.

**Revendication**

1. Procédé pour la fabrication de 3-diméthylamino-2,2-diméthylpropanal à partir d'isobutyraldéhyde, de diméthylamine et de formaldéhyde à 80–120°C, caractérisé en ce que la réaction s'effectue à un pH de 9 à 11.